# EUROPEAN PATENT APPLICATION

(11) **EP 1 003 033 A1**
(43) Date of publication of application: **24.05.2000**
(21) Application number: 99870236.9
(22) Date of filing: 16.11.1999
(51) Int. Cl.: G01N 27/327, C12Q 1/00, G01N 33/543

(54) **Sensor comprising an oligomer binding layer and method of making such sensor and arrays of such sensors**

(30) Priority: 17.11.1998 EP 98870254; 01.03.1999 US 122211 P
(71) Applicant: INTERUNIVERSITAIR MICRO-ELEKTRONICA CENTRUM VZW, 3001 Heverlee (BE); Universitaire Instituut Antwerpen, B-2610 Wilrijk (BE)
(72) Inventor: Huyberechts, Guido, 1820 Melsbroek (BE); Jordens, Sven, 3540 Herk-de-Stad (BE)
(74) Representative: Van Malderen, Joelle

(57) **Abstract**

An aim of the invention is to provide a new type of sensor, capable of recognising and/or quantifying analytes in a fluid. A further aim of the present invention is to provide such sensors with an oligomer material as a binding layer. A further aim of the present invention is to provide a novel method for the manufacture if such sensor wherein the oligomer layer is locally deposited on the sites of the sensor having a multitude of sensing sites.

## Description

### Field of the invention

The present invention relates to sensors and to methods for the making thereof.

### State of the art

Sensors and biosensors are known in the art. A biosensor can be defined as a system that can determine the existence or the concentration of a certain analyte in a sample by translating molecular recognition of said analyte ultimately into an electrical signal by means of a translation system. Biosensors can be used for any kind of analyte that can be detected by biological means. Most important is the use for detecting and/or quantifying for instance metabolites, drugs, proteins, antigen-antibody interactions, i.e. metabolite detection and affinity and immunosensing in general. For instance, detecting glucose in a diabetes patient's blood is of vital importance, detection of possibly life-threatening micro-organisms in food enhances food safety, detection and quantification of pollutants like CO, herbicides, chemicals and heavy metals are necessary to find and decontaminate polluted areas.

Biosensors can be catalogued in different groups, depending on their biological recognition system and the translation system.

Subdivision according to the biological recognition system comprises enzymatic sensors (based upon the reaction of a substrate catalysed by an enzyme, immunosensors (based upon the affinity between antibodies (or parts thereof) and antigenic determinants, e.g. ELISA test) and genosensors (based e.g. upon recognition of complementary RNA and/or DNA single strand molecules, and DNA-probes).

Subdivision of the biosensors using a classification according to the translation system used, comprises electrochemical biosensors (amperometric, potentiometric, capacitive or impedimetric), optical biosensors (e.g. Surface Plasmon Resonance (SPR), ellipsometric, fluorescence, ...), gravimetric biosensors (measuring a difference in mass by measuring a change in resonance frequency of a quartz crystal when the analyte binds or adsorbs to the crystals), and calorimetric biosensors (measuring the reaction enthalpy released when the analyte binds to a substrate).

When using recognition biomolecules, such as antibodies, enzymes, oligonucleotides or nucleic acids, these molecules need to be fixed to a carrier surface in order to be able to perform their recognising function in a reproducible way. Several possible techniques have been devised to perform the immobilisation.

One technique that is used in glucose-sensors is the immobilisation of e.g. an enzyme (glucose-oxidase) between two selectively permeable membranes. The first membrane is only permeable to the analyte (glucose), while the second membrane is only permeable to hydrogen peroxide, a reaction product of the enzyme-promoted reaction of glucose. The amount of hydrogen peroxide can easily be measured by oxidation at an electrode and correlated to the amount of analyte in the measured sample.

Another technique relies on physical adsorption to a fixed carrier surface. This has the major advantage that no special agents are necessary to bind the biomolecules to the carrier surface, the adhesion being the result of weak interactions such as Van der Waals interactions, dipole-dipole interactions and/or hydrogen bonds.

A third technique is based upon bifunctional reagents that can couple molecules to each other. Such a configuration has as a disadvantage that it can attenuate a highly active protein layer by partly shielding the activity with inert protein material. A reagent frequently used in this context is glutaraldehyde. This reagent can react with two free amino groups.

Another technique is covalent binding to a substrate. Usually, in the case of binding a protein, this implies the binding of a reagent to a free amino group that is unnecessary for the subsequent recognition of the analyte molecule. A regularly used way to perform this is to use a shielding molecule that reversibly binds to the active site of the biomolecule necessary for the analyte recognition while covalently fixing said biomolecule to the carrier substrate. Removal of the shielding molecule results in an active biosensor.

In the prior art, polymers are used to immobilise biomolecules that can recognise an analyte. The polymers are bound to an electrode surface of a sensor. Polymers can provide the necessary sites to induce adsorption by weak forces such as Van der Waals forces, dipole-dipole interactions and hydrogen bonds. Further, polymers can provide a matrix in which said biomolecules can be trapped. Polymers that are useful for this purpose are amongst many others PVA (poly(vinylalcohol)), PVC (poly(vinylchloride)), PAA (poly(acrylamide)) and PU (polyurethane). This solution provides only a slow response time, since analytes have to diffuse through the matrix to the biomolecules to be detectable.

The polymers can be deposited on one of the surfaces of the sensor by spin-coating. This method does not allow for a localized, selective deposition of the polymers. Dispensing of the polymers on localized spots of the sensor can be done but in this case the biomolecules are to be bound to the electrode prior to definition of the detection system (the sensor). After this step, heat treatments often are to follow. Such heat treatments however can destruct the biomolecules and/or the binding of the biomolecules to the polymers. The use of photolithography techniques in binding polymers to a sensor system furthermore is limited to transparent systems.

### Aims of the invention

An aim of the invention is to provide a new type of sensor, capable of recognising and/or quantifying analytes in a fluid. A further aim of the present invention is to provide such sensors with an oligomer material as a binding layer. A further aim of the present invention is to provide a novel method for the manufacture of such sensor wherein the oligomer layer is locally deposited on the sites of the sensor having a multitude of sensing sites.

### Summary of the invention

A first aspect of the present invention is to disclose a new type of oligomer material. These new oligomer materials can comprise substituted bis(styryl)benzene oligomer materials and substituted bis(styryl)thiophene oligomer materials. In the sequel of this patent application, the bis(styryl)benzene oligomer materials and the bis(styryl)thiophene oligomer materials will be denoted as backbone oligomer materials. The substituents can comprise alkoxy-groups, hydroxyl-groups, amino-groups, epoxy-groups, thiol-groups and carboxyl-groups or any other group that is capable of binding recognition molecules for detecting analytes in a fluid. These groups can be a introduced by a spacer-arm on the oligomer backbone material. The spacer-arm can be an alkane-chain or a Si-chain or a alkene-chain or derivatives.

The backbone oligomer with an alkoxy-groups can be one of the group consisting of 2,5-dimethoxy-1,4-bis(3,4,5-trimethoxystyryl)benzene, 2,5-alkoxy-1,4-bis(3,4,5-trimethoxystyryl)benzene and 1,4-bis(3,4,5-trimethoxystyryl)thiophene.

It is a second aspect of the present invention to disclose a sensor for the detection of an analyte in a fluid, characterised in that said sensor comprises:
- a substantially inert carrier substrate ;
- at least one electrode positioned on at least part of said substrate ;
- an oligomer binding layer at least partly positioned on at least part of said electrode ; and
- a recognition molecule bound to said oligomer binding layer, said biomolecule being capable of recognising said analyte.

In a preferred embodiment of the invention, the oligomer binding layer is selected from the group of substituted bis(styryl)benzene oligomer materials and substituted bis(styryl)thiopheen oligomer materials. The substituents on these backbone oligomer materials can comprise alkoxy-groups, hydroxy-groups, amino-groups, epoxy-groups, thiol-groups and carboxyl-groups or any other group that is capable of binding molecules for detecting analytes in a fluid. The oligomer layer can also be a substituted arylene alkenylene oligomer. The chemical structure of arylene alkenylene oligomers can be defined as follows: every possible sequence of one or more aromatic or heteroaromatic units like benzene, thiophene, pyrrole, aniline, indole, and many others (the arylene segments) which can be connected by one or more alkenyl segments (-(CR=CR'-)n). The arylene alkenylene oligomer can be substituted as well on the aryl segments as on the alkenyl segments.

In a preferred embodiment of the invention, the sensor is a biosensor, preferably an impedimetric biosensor which has two electrodes. The binding of the recognition molecule to the oligomer binding layer can be a covalent bonding. Said recognition molecule can be selected from the group consisting of DNA, RNA, oligonucleotide, protein, antibody, antigen and/or enzyme molecule.

The invention further concerns a sensor array comprising at least two sensors as described hereabove. Such sensor array has a multitude of sensing sites, each sensing site being a sensor. Said sites can each have a different recognition molecule and/or oligomer binding layer. The sites can also have a different number or density of recognition molecules. A number of sites can have the same recognition molecule and/or density of recognition molecule and/or have the same oligomer binding layer.

Also, the invention concerns in a third aspect the use of oligomers materials for the manufacture of biosensors.

A method for the manufacture of a sensor is disclosed, said sensor having a substantially inert carrier substrate and at least one electrode positioned on at least part of said substrate, said method comprising the following steps:
- depositing an oligomer binding layer on said electrode, and
- binding a recognition molecule capable of recognition of an analyte in a fluid to said oligomer layer.

The recognition molecule can be bound to the oligomer layer prior to the deposition step or after the step of depositing the oligomer binding layer on the electrode.

Further is disclosed a method for locally depositing an oligomer binding layer on a sensor array comprising at least two sensors. The sensors have a substantially inert carrier substrate and at least one electrode positioned on at least part of said substrate. Such sensor array has a multitude of sensing sites, each sensing site being a sensor. In a preferred embodiment, the inert carrier substrate is a common substrate of the array. Said oligomer binding layer is deposited on at least one selected electrode of said array by applying a voltage higher than the threshold voltage for electrodeposition of said oligomer on said selected electrode while the voltage applied on at least part of the other electrodes of said array is lower than said threshold voltage. In a preferred embodiment of an impedimetric biosensor array which has two electrodes for one sensing site, the oligomer binding layer can be deposited to both electrodes during this local deposition. The oligomer binding layer can be absent in-between the separate electrodes or the oligomer layer can cover the inert substrate in-between the electrodes.

Yet in another separate aspect of the invention, a sensor for the detection of an analyte in a fluid is disclosed.

The sensor according to this separate aspect of the invention comprises :
- a substantially inert carrier substrate, preferably a semiconductor substrate ;
- an oligomer binding layer in contact with a surface layer of said substrate, the oligomer binding layer comprising an oligomer with a backbone oligomer material selected from the group of substituted bis(styryl)benzene oligomer materials or substituted bis(styryl)thiopheen oligomer materials, with as substituents on these backbone oligomer materials alkoxy-groups, amino-groups, epoxy-groups, thiol-groups or carboxyl-groups, or with the oligomer being a substituted arylene alkenylene oligomer ; and
- a recognition molecule binded to said oligomer binding layer, said biomolecule being capable of recognising said analyte.

The oligomer can also consist essentially of a backbone oligomer material selected from the group of substituted bis(styryl)benzene oligomer materials or substituted bis(styryl)thiopheen oligomer materials, with as substituents on these backbone oligomer materials alkoxy-groups, amino-groups, epoxy-groups, thiol-groups or carboxyl-groups, or the oligomer can also consist essentially of a substituted arylene alkenylene oligomer. The teaching of the other aspects of the present invention, of the preferred embodiments, and of the examples and embodiments and preferred embodiments in the sequel, and any combination of aspects or embodiments or examples can be applied to this separate aspect of the invention. Therefore embodiments and examples of this separate aspect of the invention, can be construed according to the knowledge of the person skilled in the art, based on the enclosed teaching.

### Brief description of the figures

Figure 1 is representing a microphotograph of part of an embodiment of an impedimetric biosensor with two electrodes which are configured in an interdigated, separate electrode structure.

Figure 2 is representing the impedimetric biosensor of figure 1 with the detection principle.

Figure 3 is representing a backbone oligomer : 2,5-dimethoxy-1,4-bis(3,4,5-trimethoxystyryl)benzene.

Figure 4 is representing the synthesis pathway of the oligomer as described in Figure 3.

Figure 5 is describing several possibilities of functional groups which are used for the coupling of a free amino group.

Figure 6 is representing a backbone oligomer 2,5-alkoxy-1,4-bis (3,4,5-trimethoxystyryl)benzene.

Figure 7 is representing a backbone oligomer : 1,4-bis(3,4,5-trimethoxystyryl)thiophene.

Figure 8 is representing a sensor array having a 4x4 impedimetric sensor structure. According to this embodiment only line by line a voltage can be put on the sensing sites of the array. The local deposition technique of the invention in this embodiment therefore can be done only line by line.

Figure 9 is representing a flow-injection analyser using a sensor array according to the invention.

Figure 10 represents the synthesis of oligomers according to a preferred embodiment of the present invention.

Figure 11 shows the activation of the immobilised oligomers according to a preferred embodiment of the present invention.

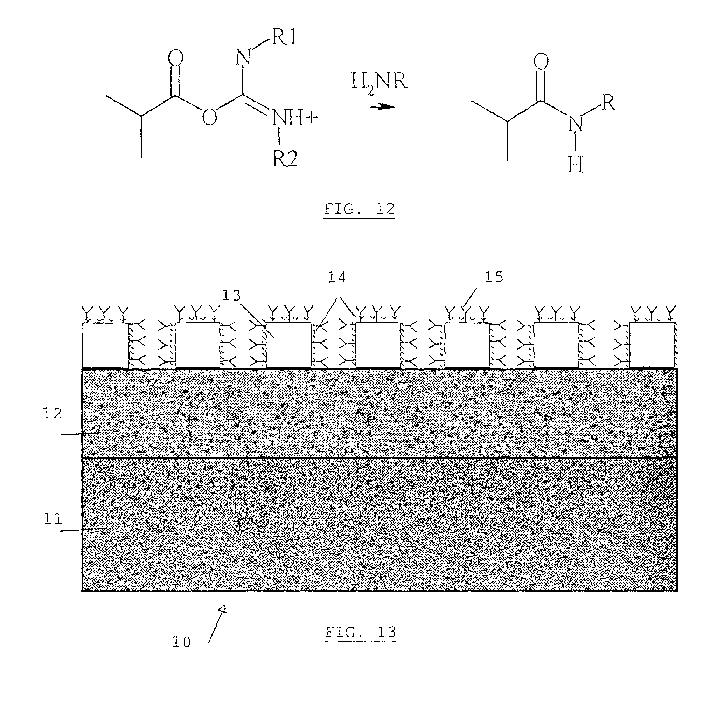

Figure 12 is representing the immobilisation of the biomolecule to the activated oligomer.

Figure 13 describes the final structure of a sensor element according to a preferred embodiment of the present invention.

### Detailed description of several embodiments of the present invention

The invention will be demonstrated using an impedimetric biosensor, as described in Van Gerwen et al., Nanoscaled interdigitated electrode arrays for biochemical sensors, Sensors and Actuators B 49 (1998) 73-80. The teaching of this document is incorporated herein by reference. This kind of sensor construction is used in all examples detailed herebelow.

The present invention can also be applied to a biosensor as described in W097/21094, incorporated herein by reference.

These impedimetric biosensors have two electrodes. Sensors with only one electrode can also be used according to the invention. Such one electrode sensors can be some gravimetric sensors or surface plasmon resonance sensors and can be fabricated. The electrode on the surface plasmon resonance sensors can be made by coating a film of gold on a prism and the oligomer layer is thereafter deposited on the gold film.

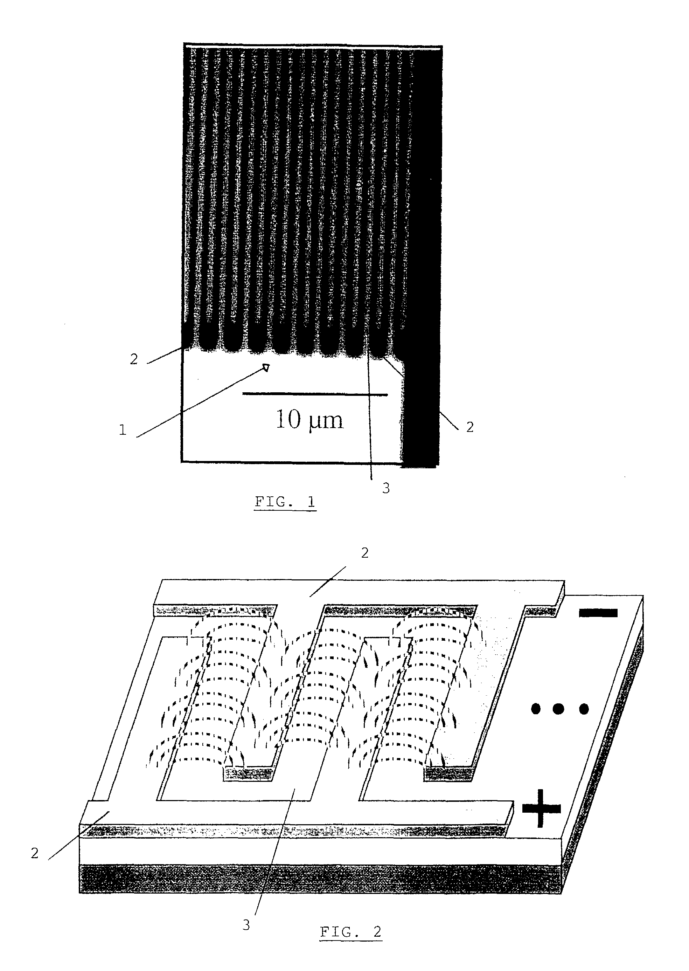

The nanoscaled interdigitated electrode arrays of Van Gerwen et al. can be realised by using deep UV lithography. Electrodes (2) have a width of between 250 and 500 nm, with siliciumdioxide (3) in between. The electrode material can comprise palladium, gold, platinum or titanium dioxide or any other electrode material that allows for electrodeposition of oligomer materials. In the case of palladium, a 50 nm layer can be deposited on a 15 nm titanium interface layer, deposited on an isolating, thermally grown silicon oxide layer. A Scanning Electronic Microscope (SEM) image is provided in Fig. 1. Preferably, and as indicated in this Fig. 1, the pattern used has a "interdigitated" layout (1).

The layout (1) of the electrodes (2) can be imposed by photolithography, followed by a lift-off procedure. An impedimetric signal can be detected when biomolecules attach to the structure and change the impedance spectrum. This impedance spectrum is measured by applying a sinusoidal alternating voltage or a combination of sinusoidal signals to the electrode structure as previously described. The resulting currents are detected with changed amplitude and phase. The current and voltage are at all times related to each other by a complex impedance. This impedance can thus be calculated at all times, and an impedance spectrum, relating the measured impedance to the applied voltage frequency. This impedance spectrum can be represented by a Bode plot, where the two components |Z| (amplitude) and ϕ (orientation) are represented in function of the voltage frequency.

Such an impedance spectrum is then mimicked by building an equivalent electronic configuration of electrical and electrochemical components. From this electronic configuration scheme, the parameter that is influenced by the binding of the analyte with the probe attached to the electrode surface, can be identified.

An embodiment of the present invention comprises the use of oligomers for binding recognition molecules to such sensors. The molecules can covalently attach to said oligomers with functional groups that are not necessary to perform the recognition function necessary to detect an analyte. Two modi operandi are possible: the recognition molecules are bound or incorporated onto the oligomers prior to the deposition step (i.e. the deposition step deposits an oligomer-biorecognition ensemble), or the biomolecules are attached to a free functional group of the oligomer after the deposition step. Thus the deposition of the oligomer can create a localised sensor element activated for binding the recognition molecule).

The use of oligomers as hereafter described has the further advantage of allowing for local deposition on the sensor. This can be exploited to provide a sensor array, with which different analytes can be measured.

### Example 1

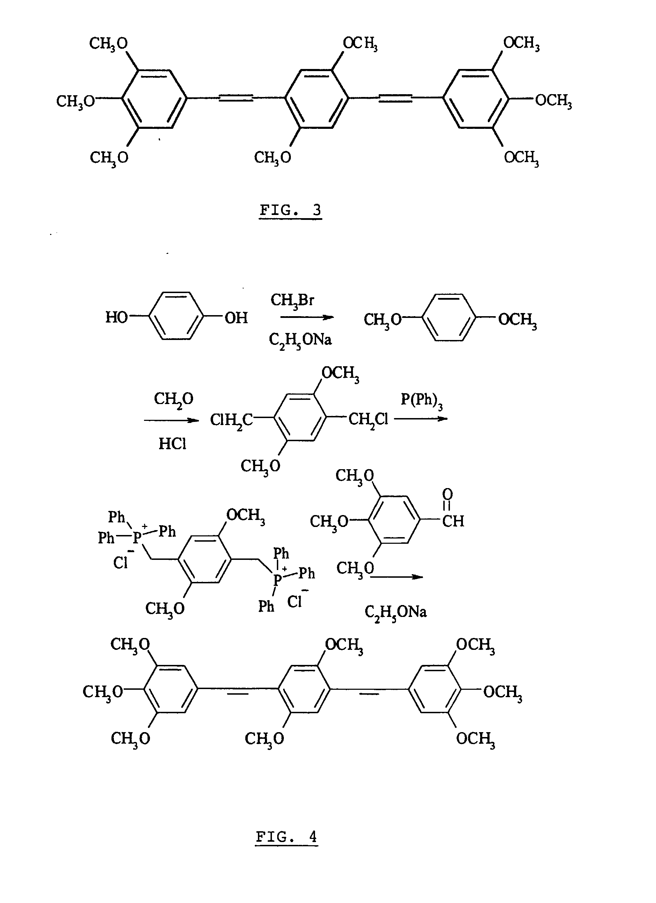

### a) Synthesis of a potential backbone oligomer 2,5-dimethoxy-1,4-bis(3,4,5-trimethoxystyryl)benzene (OMT:octamethoxytrimer) (Fig. 3)

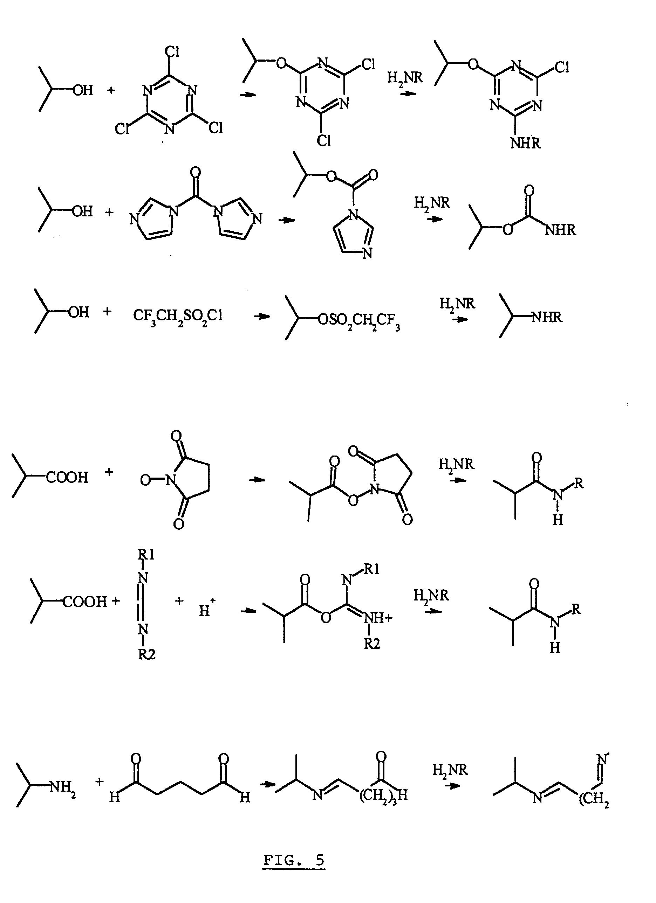

OMT can be synthesised by the Wittig route, which implies the reaction of a phosphoniumylide with a carbonylfunction to result in a double bond. The synthesis pathway is depicted in Fig. 4. Hydroquinone is reacted with methylbromide to form 1,4-dimethoxybenzene (Williamson synthesis). The 2,5-dimethoxy-1,4-dichlorobenzene results from the chloromethylation of 1,4-dimethoxybenzene. Reaction with triphenylphosphine gives rise to the according phosphonium salt. In the presence of sodiumethanolate, phosphoniumylide is formed that can further react with 3,4,5-trimethoxybenzaldehyde.

### b) Electrodeposition of the oligomer

The oligomer can be electrodeposited by applying at least a certain threshold voltage on the electrode that has to be covered. This voltage can provide the energy to deposit the oligomer on the electrode.

### c) Coupling of the biomolecule to the oligomer

Coupling of the biomolecule (that will be responsible for the recognition of the analyte) to the oligomer can be performed by using one of the available functional groups. These groups can be substituted into the oligomer by known chemical methods and are preferably a hydroxyl, amino or carboxyl group. Said groups may be substituted on the arylrings and/or on the olefine bonds.

As an example, an amino group may be included in the 3-position of the central arylring of the exemplified compounds. Furthermore, a functional group may be incorporated which is bonded to the oligomer via a spacer arm, preferably an alkane chain, which facilitates the bonding of a biomolecule. After incorporation of this functional group, this oligomer may be electrochemically deposited and thereafter the recognition biomolecule can be bonded.

The coupling of a free amino group (as in proteins and peptides) to a functional group of one of the types listed higher can be seen in Fig. 5.

### d) Recording of base impedance spectrum, mimicking said impedance spectrum by an equivalent electronic configuration and calibrating the biosensor for an analyte

### Example 2

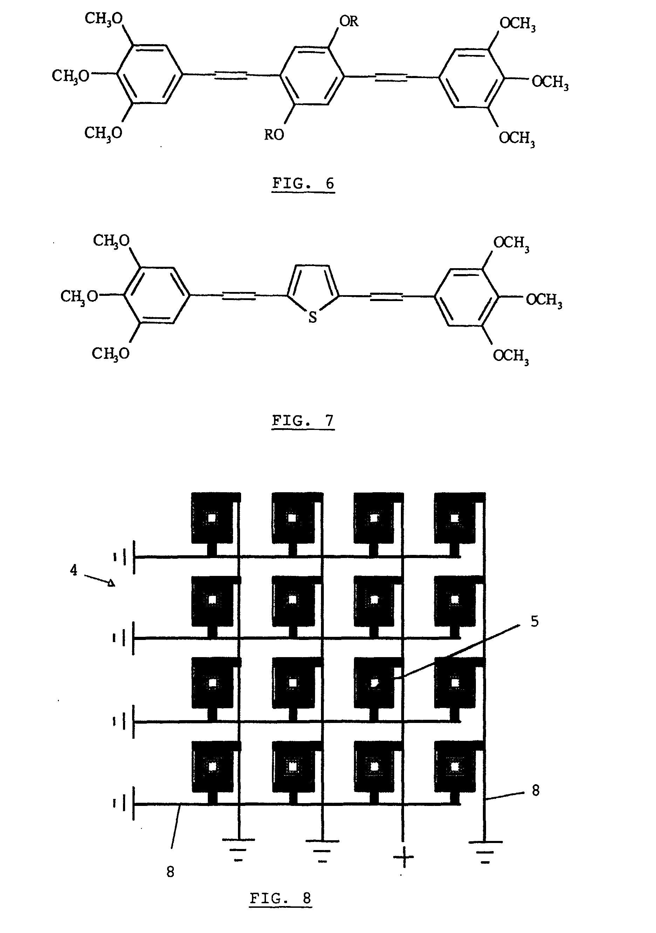

Same as example 1, but with 2,5-alkoxy-1,4-bis(3,4,5-trimethoxystyryl)benzene (Fig. 6) as backbone oligomer.

### Example 3

Same as example 1, but with 1,4-bis(3,4,5-trimethoxystyryl)thiophene (Fig. 7) as backbone oligomer.

### Example 4

Same as example 1, but with step c (attachment of the biomolecule to the oligomer) performed before step b (electrodeposition of the oligomer).

### Example 5

Same as example 2, but with step c (attachment of the biomolecule to the oligomer) performed before step b (electrodeposition of the oligomer).

### Example 6

Same as example 3, but with step c (attachment of the biomolecule to the oligomer) performed before step b (electrodeposition of the oligomer).

### Example 7

Sensor array with localised deposition (immobilisation).

A sensor array (4) (e.g. 4x4 impedimetric sensor structure such as in Fig. 8) can be selectively coated with different oligomers and/or different biomolecules or different amounts of biomolecules (to enhance the range of the sensor array for one specific analyte). This can be done by selecting one electrode (5) of the array (in the figure always one line of the array), electrodeposit only on this electrode (or line) by only applying a sufficient voltage on this electrode (or line). Specific choices of biomolecules and/or oligomers depend on the application.

### Example 8

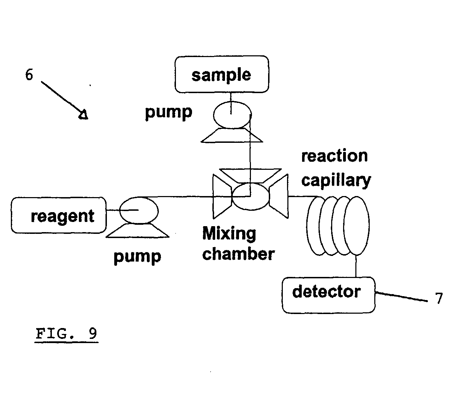

A sensor or sensor array according to the present invention can be used as a detector (7) in a flow-injection analyser (6). Such a schematic flow-injection analyser can be seen in Fig. 9.

### Example 9: Detailed Description of the Best Mode Embodiment

### Manufacturing of an electrode structure

As the carrier substrate, a silicon wafer is selected and cleaned according to well-established procedures and oxidised in order to obtain an insulating layer of oxide on the silicon. Alternatively other insulating layers such as e.g. silicon nitride can be used in order to deposit or grow an insulating material on the silicon substrate. Alternatively ceramic or plastic insulating substrates can be used, given a sufficient surface smoothness and other relevant properties.

A metal layer is deposited on the insulating substrate and patterned according to techniques known in the manufacturing of electronic circuits, i.e. patterning can be performed by a lift-off process or by ething, following a photolithographic definition step, resp. prior or after the metal deposition step. In general anchoring or adhesion layers are deposited prior to the deposition of the metal layer in order to obtain satisfactory adhesion of the metal layer on the insulating substrate. Anchoring layers of Ti and Ti/Pd multilayers are used, followed by the deposition of resp. Pd and Au metal layers. After patterning the insulating substrate is covered by an array of metal electrodes, which can each individually be addressed for deposition or read-out as further explained.

The silicon wafer is diced into sensor arrays, each containing a limited number of electrode structures as defined by the actual lay-out as defined by the photolithographical patterning and design required by the application.

Each die, containing a sensor array, is packaged in such way that the electrodes can be contacted by electrical means to the outside world and such that on immersion in solution only a predefined area of the sensor array, i.e. the electrode ensembles are allowed to contact the solution (and not the wires or contact lines not involved in the electrode structure itself).

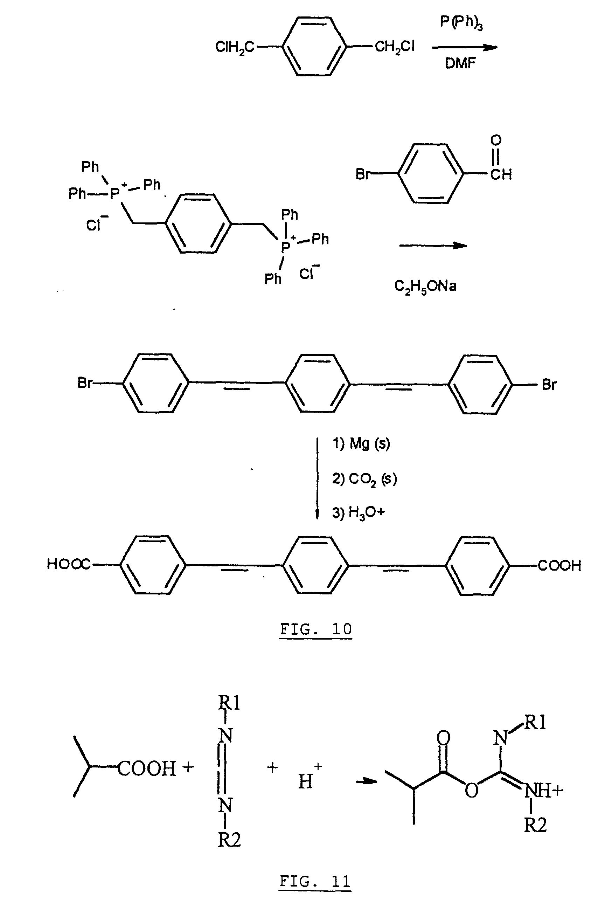

### Synthesis of oligomers : figure 10

In a first step one forms the difosfoniumsalt. This is done by using dimethylformamide as a solvent. The reaction takes 5-10 hrs under constant reflux temperature. In a final step the precipitate is washed with ether.

For the Wittig reaction one can use dry ethanol as a solvent. To this mixture, one adds sodiumethanolate as the base. This reaction takes 5-10 hrs at a temperature between room and reflux. Everything is done in ultra dry conditions and under N₂-protection to avoid secondary reactions of the Grignard-reagent with oxygen and water.

For the Grignard reaction one uses dry ether as a solvent. In this solvent one brings an amount of solid magnesium together with a catalyst: I₂. To this mixture one adds the dibromo-molecule and one lets it react. When everything is dissolved, one adds the solid CO₂. In a final step one works it up with HCl and water.

### Deposition of the oligomer on an electrode structure

An oligomer in acetonitrile solution is brought in the presence of 0.1 M LiClO₄. One applies a potential greater than the oxidation potential of the oligomer over the sensor element and a reference electrode. In this way, one is depositing an oligomer film on the electrodes of the sensor or sensor element (a sensing site). Since a potential is applied over only one sensor element of the whole array and a reference electrode, the deposition of the oligomer is limited to this sensor element. In this way localised deposition of the oligomer layer is achieved.

### Modification of the immobilized oligomer

In this step, the carboxyl-function of the oligomer is activated by using a carbodiimide (EDC: 1-Ethyl-3-(3-Dimethylaminopropyl) Carbodiimide), In this way, one gets much better coupling yields between the oligomer and the biomolecule.

### Deposition of a biomolecule on the immobilized oligomer layer

A solution containing the biomolecule to be deposited is flushed over the sensor array with a single sensor (sensing site) or a limited number of sensor elements (sensing sites) covered by the activated oligomer. Sufficient time is allowed to complete the interaction between the biomolecule and the anchoring sites of the oligomer layer. The solution containing the biomolecule is washed away and the molecules not covalently bound to the oligomer surface are removed, leaving a single sensor element with immobilised oligomer-biomolecule combination on the surface.

### Structure of the sensor element:

In figure 13 (not to scale) the sensor element (10) which is build up by different layers is shown. The base layer in Si (11) is covered by an insulating SiO₂ layer (12). The electrodes in Pd (13) are covered with oligomer material (14), which is coupled with biomolecules (15).

### Array formation

Steps 3 and 4 are repeated as many times as there are sensor elements to be covered in the sensor array. Step 4 is repeated with each time a different biomolecule, the selection of which is dictated by the actual application the sensor array is intended for. Step 3 is in general the same for all repetitions if the chemical coupling mechanism for the covalent binding is the same for all biomolecules. Eventually the nature of the oligomer in step 3 is to be adapted depending on the nature of the biomolecule in order to result in a perfect binding.

### Testing

The sensor array is brought into contact with a solution potentially containing the analytes of interest, the solution being obtained either with or without sample preparation steps, such as filtration or pre-concentration. In the case of the envisaged application, where the sensor array is constituted from a number of interdigitated electrode structures, an impedimetric analysis is performed in order to obtain the impedance spectra of the individual sensor elements. Given the application the detection and/or quantification of the analytes can be performed based on the detection of quantitative parameters from the impedance spectrum obtained from either a single frequency or from a combination of frequencies.

## Claims

1. A sensor for the detection of an analyte in a fluid, said sensor comprising :
- a substantially inert carrier substrate ;
- at least one electrode positioned on at least part of said substrate ;
- an oligomer binding layer at least partly positioned on at least part of said electrode ; and
- a recognition molecule bound to said oligomer binding layer, said biomolecule being capable of recognising said analyte.

2. The sensor as recited in claim 1 wherein the oligomer binding layer is a substituted backbone oligomer material.

3. The sensor as recited in claim 2 wherein the substituents on the backbone oligomer materials comprise at least one of the group of alkoxy-groups, hydroxy-groups, amino-groups, epoxy-groups, thiol-groups and carboxyl-groups or any other group that is capable of binding recognition molecules for detecting analytes in a fluid.

4. The sensor as recited in claim 1 wherein the oligomer binding layer is a substituted arylene alkenylene oligomer.

5. Sensor as in any one of the preceding claims, wherein the oligomer layer is selected from the group consisting of 2,5-dimethoxy-1,4-bis(3,4,5-trimethoxystyryl)benzene, 2,5-alkoxy-1,4-bis(3,4,5-trimethoxystyryl)benzene and/or 1,4-bis(3,4,5-trimethoxystyryl),thiophene.

6. Sensor as recited in claim 1, wherein the binding of the analyte to the oligomer layer is a covalent bonding.

7. Sensor as in any one of the preceding claims, wherein said recognition molecule is selected from the group consisting of DNA, RNA, protein, antibody, antigen, oligonucleotide and enzyme molecules.

8. Sensor array comprising at least two sensors according to any one of the preceding claims, wherein said sensors have a different recognition molecule and/or oligomer layer.

9. A method for manufacturing a sensor, said sensor having a substantially inert carrier substrate and at least one electrode positioned on at least part of said substrate, said method comprising the steps of :
- depositing an oligomer binding layer on said electrode, and
- binding a recognition molecule capable of recognition of an analyte in a fluid to said oligomer layer.

10. The method as recited in claim 9 wherein the recognition molecule is bound to the oligomer layer prior to the step of depositing the oligomer binding layer on said electrode.

11. The method as recited in claim 9 wherein the recognition molecule is bound to the oligomer layer after the step of depositing the oligomer binding layer on the electrode.

12. A method for locally depositing an oligomer binding layer on a sensor array comprising at least two sensors, the sensors having a substantially inert carrier substrate and at least one electrode positioned on at least part of said substrate, Said oligomer binding layer being deposited on at least one selected electrode of said array by applying a voltage higher than the threshold voltage for electrodeposition of said oligomer on said selected electrode while the voltage applied on at least part of the other electrodes of said array is lower than said threshold voltage.

13. Method for selectively depositing an oligomer layer on an electrode array structure, wherein said oligomer layer is deposited on a selected electrode of said electrode array by applying a voltage higher than the threshold voltage for electrodeposition of said oligomer over said selected electrode while the voltage applied over the other electrodes of said electrode array is lower than said threshold voltage.

14. The method as recited in claim 12 and 13 wherein the oligomer binding layer is a substituted backbone oligomer material.

15. The sensor as recited in claim 12 and 13 wherein the substituents on the backbone oligomer materials comprise at least one of the group of alkoxy-groups, hydroxyl-groups, amino-groups, epoxy-groups, thiol-groups and carboxyl-groups or any other group that is capable of binding molecules for detecting analytes in a fluid.

16. The sensor as recited in claim 12 and 13 wherein the oligomer layer is a substituted arylene alkenylene oligomer.

17. Sensor as in claims 12 and 13, wherein the oligomer layer is selected from the group consisting of 2,5-dimethoxy-1,4-bis (3,4,5-trimethoxystyryl)benzene, 2,5-alkoxy-1,4-bis(3,4,5-trimethoxystyryl)benzene and/or 1,4-bis(3,4,5-trimethoxystyryl)thiophene.

18. An oligomer material being a substituted bis(styryl)benzene oligomer materials or a substituted bis(styryl)thiophene oligomer materials.

19. The material as recited in claim 18 wherein the substituents are at least one of the group of alkoxy-groups, hydroxyl-groups, amino-groups, epoxy-groups, thiol-groups and carboxyl-groups or any other group that is capable of binding molecules for detecting analytes in a fluid.
